# EUROPEAN PATENT APPLICATION

(11) **EP 4 725 504 A1**
(43) Date of publication of application: **15.04.2026**
(21) Application number: 24206471.5
(22) Date of filing: 14.10.2024
(51) Int. Cl.: A61K 41/00, A61K 47/61, A61K 47/69, A61P 35/00

(54) **POLYMERIC PRODRUG NANOPARTICLE FOR TUMOR-TARGETED ULTRASOUND-ACTIVATED CANCER THERAPY**

(71) Applicant: Schleuning, Christian, 22851 Norderstedt (DE)
(72) Inventor: Karges, Johannes, 44791 Bochum (DE)
(74) Representative: Stork Bamberger Patentanwälte PartmbB

(57) **Abstract**

A polymeric prodrug nanoparticle for the targeted accumulation and activation in tumor tissue, consisting of the following monomers:
a) at least one platinum(IV) complex and
b) at least one sonosensitizer, and
c) a compound having a hydrophilic group providing this hydrophilic group at each terminal end of the prodrug nanoparticle, wherein a), b) and c) are each covalently bonded to one another by means of a linker and a) and b) are arranged stochastically distributed within the polymeric prodrug nanoparticle can be used to treat tumors and in particular metastases in a way that is well tolerated by the body. Upon irradiation of the polymeric prodrug nanoparticle with ultrasound, the platinum(IV) prodrug is activated in the tumor region, resulting in the release of cytotoxic platinum and killing of tumor cells.

## Description

The present invention relates to a prodrug nanoparticle for tumor-targeted ultrasound-activated cancer therapy. The invention further relates to a formulation, a use of the prodrug nanoparticle and its preparation method.

Cancer is one of the most common causes of disease-related death worldwide and refers in particular to the development of a malignant neoplasm. Both, the uncontrolled and increased proliferation of cells and the inhibition of naturally occurring apoptosis, are due to various mutations or the deletion of genes. Malignant tumors can occur in various organs of the body and develop from different cell types. The tumor cells have invasive and migratory potential, allowing them to migrate into the healthy tissue and increasingly lead to its destruction. Single tumor cells or tumor cell clusters can also detach from the primary tumor and thus form local metastases, regional metastases or distant metastases via various metastasis pathways. The high mortality rate is largely due to metastatic processes. How frequently the different types of cancer occur in the population depends, among other things, on the age, gender and territorial region of residence of the affected patients.

In many tumor entities, at the time of diagnosis, metastases have already occurred in other tissues or organs, such as the liver, the lungs, the bones, the brain or the lymph nodes. In addition to resection of the primary tumor and radiotherapy, chemotherapy one of the standard treatment options. During chemotherapy, cytostatic drugs are administered to the patient. Cytostatic drugs, however, are poorly tolerated and impose great strain on the body. Cytostatic drugs not only target tumor cells, but also healthy cells. Highly proliferating cells such as hair follicle cells and mucosal epithelial cells are particularly affected. This can lead to frequent side effects such as hair loss, nausea, vomiting and serious side effects such as damage to peripheral sensory nerves or damage to blood formation with susceptibility to infections of all kinds, anemia or coagulation disorders. The insufficient accumulation of cytostatic drugs in tumor tissue represents a further limitation to a successful and effective treatability of tumors. Cytostatic drugs are cytotoxic chemical substances that interfere with the cell cycle and inhibit cell growth by restricting the cells' ability to divide. Platinum(II) complexes, in particular cisplatin, oxaliplatin and carboplatin, are among the most frequently used cytostatic drugs in cancer therapy. These compounds are administered in more than 50 % of all chemotherapeutic treatments worldwide. The cytostatic drugs must be administered in high doses in order to obtain a sufficient concentration in the tumor to be treated, resulting in the undesirable side effects described above.

It has been found that these disadvantages can be reduced if the platinum(ll) complexes are encapsulated in nanoparticles, resulting in a better selective accumulation of the platinum(II) complex in the tumor tissue. Due to the high proliferation rate of the tumor cells and the need for their own vascular system, intratumoral blood vessels are formed. Compared to normal tissue, intratumoral blood vessels often exhibit increased permeability of the capillary endothelium for larger particles. Due to the so-called Enhanced Permeability and Retention (EPR) effect, the platinum(II) complexes encapsulated in nanoparticles enter into the tumor tissue and selectively accumulate there, while the possibility for the nanoparticles to move into healthy tissue is greatly reduced. In addition, the platinum(II) complexes are protected from direct excretion via e. g. the kidneys by encapsulation in nanoparticles and are present in high concentrations until they reach the target site.

In order to reduce the side effects of cytostatic drugs, an alternative option is not to administer them to the body in their medically effective form, but to administer them as a precursor in the form of a prodrug. In the case of platinum, for example, it is not administered as platinum(II), but in the therapeutically inactive form of platinum(IV).

Photodynamic therapy (PDT) is a method that is often used to treat tumors. This non-invasive treatment method uses light in combination with a photosensitizer. A photosensitizer is a light-sensitive substance that triggers a reaction in the tissue when irradiated with light of a suitable wavelength. PDT is a particularly promising treatment option for superficial or easily reachable tumors, such as tumors of the skin, larynx or oral cavity.

For example, "Wei D, Huang Y, Wang B, Ma L, Karges J, Xiao H. Photo-Reduction with NIR Light of Nucleus-Targeting PtIV Nanoparticles for Combined Tumor-Targeted Chemotherapy and Photodynamic Immunotherapy. Angew Chem Int Ed Engl. 2022 May 9;61(20):e202201486. doi: 10.1002/anie.202201486. Epub 2022 Mar 16. PMID: 35212437." discloses an oxaliplatin(IV) prodrug that can be reduced to the therapeutically active oxaliplatin(II) form by near infrared light in the tumor microenvironment. For this purpose, a photosensitizer with an aggregation-induced emission (AIE) group is used. To achieve increased accumulation in the nucleus of the tumor cells, nucleus-specific peptides were used in this study. All these components are covalently linked inside a polymer which is able to self-assemble into nanoparticles. However, as a fundamental prerequisite, these nanoparticles would have to first selectively reach the tumor tissue in order to ensure specific accumulation in the nucleus. The selective accumulation of the oxaliplatin(IV) prodrug in the tumor tissue is based exclusively on the passive EPR effect, so that diffusion of the nanoparticles is not readily possible if the tumor's blood vessels are intact. Even if EPR takes place, it does not ensure uptake in the nucleus but only in the tumorous tissue.

It is therefore an object of the present invention to create a stable prodrug nanoparticle that is well tolerated by the body, is inactive in healthy tissue and can be selectively activated in tumor tissue. Furthermore, it is an object of the invention to provide a prodrug nanoparticle which can also be activated in deep-seated tumors without invasive intervention. In particular, it is also an object of the invention to create a chemical functionalization that ensures reliable and selective accumulation of a prodrug nanoparticle in tumor tissue. In addition, it is an object of the present invention to propose a curative or palliative therapy for the treatment of a malignant tumor or its metastases. Furthermore, it is an object of the present invention to provide a formulation of such a prodrug nanoparticle, its use and preparation method.

The object is achieved by a polymeric prodrug nanoparticle for targeted accumulation and activation in tumor tissue, consisting of the following monomers:
a) at least one platinum(IV) complex and
b) at least one sonosensitizer,
   and
c) a compound having a hydrophilic group providing this hydrophilic group at each terminal end of the polymeric prodrug nanoparticle,
   characterized in that
a), b) and c) are each covalently bonded to one another by means of a linker and a) and b) are arranged stochastically distributed within the polymeric prodrug nanoparticle.

Surprisingly, it has been found that the polymeric prodrug nanoparticle according to the invention can be used to treat tumors and in particular metastases in a way that is well tolerated by the body. The polymeric prodrug nanoparticles according to the invention are particularly suitable for the treatment of carcinomas, sarcomas, lymphomas, melanomas and germ cell tumors, and their respective metastases. The polymeric prodrug nanoparticles accumulate selectively in a tumor and its metastases after administration e. g. via intravenous injection. After irradiation with ultrasound, the platinum(IV) prodrug is activated in the tumor region, resulting in the release of cytotoxic platinum and killing of tumor cells. Platinum(II) has a cytotoxic effect on cells. The cytotoxic effect is based on the inhibition of DNA replication by cross-linking two neighboring guanine bases of a DNA strand. This disrupts the structure of the DNA, rendering it non-functional. Cell metabolism comes to a standstill and the cell initiates apoptosis.

Metastases in the lungs or liver, but also other regionally limited metastases, even if they are localized deeper in the body, can be treated particularly well with the polymeric prodrug nanoparticles according to the invention. In other words, the present invention provides for a nanoformulated platinum-prodrug for spatially selective, ultrasound-activated anticancer therapy.

Preferably, the cytotoxic platinum is a platinum(II) complex with four ligands, which are independently selected from the group consisting of ammonia, hydroxide, halide, particularly chloride, oxalate, diamines, dicarboxylate and glycolate, optionally two of the ligands are linked to form a first bidentate ligand or two times two ligands are linked to form a first and a second bidentate ligand. Particularly preferred, the cytotoxic platinum is cisplatin, oxaliplatin, carboplatin, nedaplatin, lobaplatin or heptaplatin.

The platinum(IV) complex is complexed with two carbamate ligands in addition to the ligands complexed with the respective platinum drug (e. g. oxalate and 1,2-diaminocyclohexane for oxaliplatin or two chloride and two ammonia ligands for cisplatin). The at least one platinum(IV) complex can be connected to another platinum(IV) complex or to a sonosensitizer by a linker. The sequence in which platinum(IV) complexes and sonosensitizers are bonded to each other via the linker is determined by stochastic distribution, i. e. the platinum (IV) complexes and the sonosensitizers are arranged stochastically distributed within the polymeric prodrug nanoparticle. Particularly preferably, the two carbamate ligands are the same carbamates; however, different carbamate ligands can also be used.

The compound having a hydrophilic group providing this hydrophilic group at each terminal end of the molecule leads to self-assembly into a nanoparticle structure. The compounds having a hydrophilic group are bonded to the polymeric prodrug nanoparticle molecule in such a way that there is a hydrophilic group at each terminal end of the polymeric prodrug nanoparticle, wherein the polymeric prodrug nanoparticle regularly disposes of two terminal ends. Components a), b) and c) are covalently bonded to each other in one molecule. As a result, the platinum(IV) complex is stable and protected from e. g. direct excretion via the kidneys. Another advantage is that high concentrations of the drug can be used.

Due to the covalent bond of the respective components the at least one inactive platinum(IV) complex a) and the at least one sonosensitizer b) in the polymeric prodrug nanoparticle are in close proximity to each other. This ensures spatial and temporal control over the activation to a therapeutic platinum(II) complex form. The sonosensitizer enables activation by ultrasound and is therefore an "ultrasound antenna". The ultrasound radiation penetrates into deep tissue and also enables reliable reduction of the platinum(IV) complex there. Due to the EPR effect, the polymeric prodrug nanoparticles selectively accumulate in the tumor microenvironment. In other words, the polymeric prodrug nanoparticles accumulate predominantly in the tumor tissue and only slightly in healthy tissue. Advantageously, the tumor region is selectively irradiated using ultrasound. In healthy tissue even if the polymeric prodrug nanoparticles have accumulated there the polymeric prodrug nanoparticles remain inactive. In tumor tissue, however, the platinum(IV) prodrug is activated by the ultrasound treatment and the platinum(IV) in the platinum drug complex derivative is reduced to platinum(II) by splitting off the two carbamate ligands. This process generates the platinum(II) drug, the pharmacologically active compound, which provides for the targeted treatment of tumors and/or metastases.

The polymeric prodrug nanoparticle of the present invention can be used to treat both, primary tumors and, in particular, metastases. Metastasis refers to the detachment of tumor cells from the primary tumor and their invasion and migration into other tissues or organs. The resulting tumors nodules are called metastases. While the primary tumor can be surgically removed in many cases, this is often not possible or only possible with difficulty in the case of metastases. The prodrug of the present invention is excellently suited for the treatment of metastases.

While the prodrug remains stable under physiological conditions, it is reduced in the presence of the sonosensitizer and under the influence of ultrasound radiation. Thus, the prodrug according to the invention remains stable and therefore inactive in healthy tissue but is rapidly reduced to the therapeutically active analog compounds in cancerous tissue upon ultrasound irradiation. In contrast to the previously performed chemical reduction of the prodrug by intracellular reducing agents (e.g. glutathione, ascorbic acid, metallothionein, thioredoxin), which are present in high concentrations in cancer cells, but also in healthy cells, the activation mechanism according to the invention is associated with good spatial and temporal control and a high reduction efficiency.

A preferred embodiment is characterized in that the at least one platinum(IV) complex is a platinum(IV) derivative of a platinum(II) complex with four ligands, which are independently selected from the group consisting of ammonia, hydroxide, halide, particularly chloride, oxalate, diamines, dicarboxylate and glycolate, optionally two of the ligands are linked to form a first bidentate ligand or two times two ligands are linked to form a second bidentate ligand.

Particularly preferred, the platinum (IV) is a platinum(IV) derivative of cisplatin, oxaliplatin, carboplatin, nedaplatin, lobaplatin or heptaplatin, wherein cisplatin, oxaliplatin and carboplatin are particularly preferred.

The platinum(ll) drug and the corresponding platinum(IV) complex precursor, respectively, can be selected according to the tumor type to be treated. Cisplatin is a cytostatic drug that is used to treat many different types of cancer such as testicular, ovarian, urinary bladder, squamous cell and small cell lung cancer. Oxaliplatin is particularly suitable for the treatment of colorectal carcinoma. Furthermore, Oxaliplatin is able to trigger immunogenic cell death. In addition to its direct effects on the tumor cells, oxaliplatin activates the immune system so that tumors and metastases are identified and eliminated by immune cells. Carboplatin is typically used to treat ovarian and bronchial carcinoma. It is also possible to use other specific platinum(IV) complexes. In certain tumor diseases, these other platinum(IV) complexes are particularly suitable and can be integrated into the polymeric prodrug nanoparticles. These platinum(IV) complexes include the platinum(IV) derivative of nedaplatin for the treatment of nasopharyngeal carcinoma, the platinum(IV) derivative of lobaplatin for the treatment of small cell lung cancer, inoperable metastatic breast cancer and chronic myelogenous leukemia and the platinum(IV) derivative of heptaplatin for the treatment of gastric cancer. In addition, further platinum(IV) complexes can also be integrated into the polymeric prodrug nanoparticles and be used for therapy. This is particularly interesting for tumors with known cisplatin resistance, which are difficult to be treated effectively.

Furthermore, it is possible that two or more different platinum(IV) complexes are bonded into the polymeric prodrug nanoparticle. Particularly preferable, however, only one type of platinum(IV) complex is bonded into the polymeric prodrug nanoparticle.

In a particularly preferred embodiment the general chemical structure of the polymeric product nanoparticle is a compound of the formula: 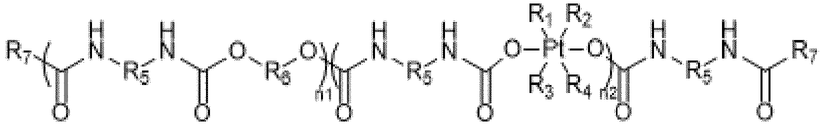 wherein:
R₁, R₂, R₃ and R₄ are independently selected from the group consisting of ammonia, hydroxide, halide, particularly chloride, oxalate, diamines, dicarboxylate and glycolate,
optionally R₁ and R₂ are linked to form a first bidentate ligand, and/or R₃ and R₄ are linked to form a second bidentate ligand;
R₅ is the linker
R₆ is b), i. e. the at least one sonosensitizer,
R₇ is c), i. e. the compound having a hydrophilic group providing this hydrophilic group at each terminal end of the polymeric prodrug nanoparticle and
n₁, n₂ are 1 to 50, particularly 1 to 10, preferably 3 to 8.

A particularly preferred embodiment is characterized in that the sonosensitizer is a cyanine or a tetrapyrrole or a metal complex of a cyanine or a tetrapyrrole, preferably a heme or a heme compound, in particular selected from the group consisting of hemoglobin, hemin b and hemin a. Other particularly preferred tetrapyrroles are porphyrins, chlorins, bacteriochlorins and phthalocyanins as well as the metal complexes thereof.

Optionally, the sonosensitizer contains one or particularly two spacer molecules, which serve to better bond the sonosensitizer to the linker. The spacer molecules are unbranched or branched alkyls or aryls with 1 to 10 carbon atoms and optionally ester, amid, carbamate, hydroxy, amine, carboxyle and/or carbonyle functions.

Sonosensitizers such as cyanines or tetrapyrroles, serve as a molecular ultrasound antenna. Advantageously, the immediate proximity of the sonosensitizer to the platinum(IV) complex result in a particularly good activation upon ultrasonic irradiation. This activation results in a reduction of the platinum(IV) complex to a medically active platin(II) complex with cleavage of the two carbamate ligands.

Furthermore, it is possible that two or more different sonosensitizer are bonded into the polymeric prodrug nanoparticle. Particularly preferable, however, only one type of sonosensitizer is bonded into the polymeric prodrug nanoparticle.

The linker has at least two, preferably exactly two, terminal ends. A preferred embodiment is characterized in that the linker is a diisocyanate. Diisocyanates are organic compounds that contain two terminal isocyanate groups and can be synthesized easily and split off as required. Diisocyanates are highly reactive in water at room temperature with components a), b) and c) and render covalently bonded carbamates.

A particularly preferred embodiment is characterized in that the linker has a branched or unbranched alkyl radical between the functional groups, the main chain of the alkyl radical having three to ten carbon atoms, preferably four to seven carbon atoms. The side chains preferably comprise one to ten carbon atoms and may be linear or cyclic aliphatic or arylic group.

Linear alkyls are particularly preferred. The alkyl chain - main chain and/or side chain - may have one or more substituents that - apart from containing carbon and hydrogen - may contain one or more of oxygen, nitrogen, sulfur and phosphor as heteroatoms. Particularly preferred substituents are ester, amid, carbamate, hydroxy, amine, carboxyle, carbonyle and thioether groups, ester, amid and carbamate groups being particularly preferred.

It is particularly preferable for the main chain of the alkyl radicals to have five carbon atoms. This ensures that the junction of the sonosensitizer, particularly if porphyrine is used as a sonosensitizer, with the platinum(IV) complex runs efficiently, that the preparation is trouble-free and that an efficient energy transfer is ensured to reduce the platinum(IV) and to split off the carbamate ligands.

A preferred embodiment is characterized in that the compounds having a hydrophilic group are PEG groups, peptides, proteins, antibodies, aptamers or small molecules.

This is a particularly preferable way to ensure that the polymeric prodrug nanoparticles remain in the hydrophilic tumor tissue or tumor microenvironment to allow for the activation by ultrasound in due course.

The hydrophilic groups provide for the polymer forming a self-assembling nanoparticle structure.

Peptides consist of short chains of amino acids that are bonded by peptide bonds. In a preferred embodiment of the present invention the peptide is a poly amino acid of one type of amino acid, polyarginine, in particular octaarginine, being particularly preferred. Preferably the peptide consists of 1 to 100 amino acids, 5 to 15 amino acids being particularly preferred.

Antibodies bind specifically to an epitope of an antigen expressed on the surface.

An aptamer is a short single-stranded DNA or RNA oligonucleotide with a length of 25 - 70 bases. The special feature of this oligonucleotide is that it can bind a specific molecule by means of its 3D structure. The properties of aptamers are to a certain extent a mixture of those of small molecules and antibodies. This results in a very favorable combination. The properties are very high chemical stability, low immunogenicity, high specificity and affinity and the ability to specifically influence protein-protein interactions. Furthermore, aptamers are not biologically produced but chemically synthesized. This makes the production of aptamers considerably cheaper. Peptides, antibodies and aptamers have the specific task of binding to surface structures and anchoring the polymeric prodrug nanoparticle there.

A small molecule (or metabolite) is a low molecular weight organic compound, typically involved in a biological process as a substrate or product. Small molecules are molecules within a mass range of 50 - 1500 daltons (Da) and include sugars, lipids, amino acids, fatty acids, phenolic compounds or alkaloids.

In addition, peptides, proteins, antibodies, aptamers or small molecules can inhibit specific surface molecules resulting in their limited function in tumor progression.

A further preferred embodiment of the present invention is characterized in that the compounds having a hydrophilic group are tumor antigen-binding or tumor-associated antigen-binding domains.

The specification of the hydrophilic groups provides them with a functional property. This functionalization reliably ensures that the polymeric prodrug nanoparticle binds in the vicinity of the tumor, in particular in the tumor microenvironment. The tumor antigen, to which the tumor antigen-binding domain binds, is expressed on the tumor cells, and the tumor-associated antigen, to which the tumor-associated antigen-binding domain binds, is expressed on the cells of the tumor microenvironment. This has the advantage that the polymeric prodrug nanoparticle selectively accumulates in the tumor tissue and only to a small extent in healthy tissue.

A further advantage is that this ensures active and selective accumulation in the tumor tissue. This additionally increases the selective accumulation in the tumor tissue due to the passive EPR effect. This also ensures that the polymeric prodrug nanoparticle remains accumulated in the tumor tissue over a longer period of time.

A particular preferred embodiment is characterized in that the tumor-associated antigen-binding domains are directed against VEGFR2. VEGFR2 is overexpressed on the endothelial cells of the intratumoral blood vessels compared to the endothelial cells outside the tumor microenvironment. The endothelial cells represent the first contact with the prodrug nanoparticle. By targeting the tumor-associated antigen-binding domain against VEGFR2, it is ensured that the polymeric prodrug nanoparticle binds predominantly in the direct tumor microenvironment. In addition to the passive EPR effect, this promotes accumulation within the tumor tissue. Another advantage is that VEGFR2 is temporarily blocked and thus the VEGF/VEGFR signal transduction pathway is inhibited, which reduces the formation of new blood vessels (neoangiogenesis) and thus tumor progression. This is particularly important in the progression of colorectal carcinoma, mammary carcinoma, pancreatic carcinoma, prostate carcinoma, bronchial carcinoma, endometrial carcinoma, non-Hodgkin's lymphoma, malignant mesothelioma and germ cell tumors.

Further preferably, the tumor-associated antigen-binding domain is directed against EGFR. The EGF signaling pathway is permanently activated, particularly in tumors with a mutation of the KRAS oncogene, which leads to a high proliferation rate of the tumor cells. Thus, there is a significant overexpression of EGFR on the surface of the tumor cells compared to healthy cells. Targeting the EGFR therefore not only directly targets the tumor cells, but also inhibits further cell growth. This targeting can be achieved using an antibody, such as Cetuximab or Panitumab.

Preferably, the tumor-associated antigen-binding domain is directed against CD137. CD137 is expressed in particular on the cell surface of immune cells in the tumor microenvironment, which increasingly colonize there. Thus, the polymeric prodrug nanoparticle binds in close proximity to the tumor cells. Another effect is that this additionally stimulates the immune system.

Further preferably, the tumor-associated antigen-binding domain can prevent resistance to chemotherapeutics. This is achieved by targeting inhibition of multidrug membrane transporters such as ABCG2 using Verapamil.

In a further preferred embodiment of the present invention, the polymeric prodrug nanoparticle is additionally loaded with at least one of at least one further antitumoral substance and at least one drug transporter inhibitor.

The further antitumoral substance and/or drug transporter inhibitor is/are physically encapsulated in the polymeric prodrug nanoparticle. Unlike the monomers making up the polymeric prodrug nanoparticle, the further antitumoral substance and/or drug transporter inhibitor is/are not covalently bonded, but is/are loaded due to hydrophilic interaction.

A drug transporter inhibitor is a compound effecting that the drug remains intercellular, i. e. preventing the drug from being exported from the cell. Examples of such drug transporter inhibitors are efflux pump inhibitors.

The additional loading of the nanoparticles with at least one further antitumoral substance extends the monotherapy to a combination therapy. The effect of the treatment is thus increased by synergistic effects of different antitumoral substances. In particular, the nanoparticles are preferably loaded with antitumoral substances that act through different mechanisms. Combination therapy reduces the likelihood of resistant tumor cells developing. Likewise, by combining the advantages of cytostatic combination therapy with the advantages of prodrug nanoparticles (selective accumulation and activation in the tumor), increased local anti-tumor activity is achieved with reduced systemic side effects. This makes a curative therapeutic approach possible in situations that were previously only accessible to palliative therapy (e.g. multiple or non-resectable liver metastases).

In a further preferred embodiment of the invention the nanoparticles are additionally loaded with 5-fluorouracil and folinic acid, in particular analogous to the FOLFOX chemotherapy regimen. FOLFOX refers to a chemotherapeutic treatment regimen for colorectal carcinoma, consisting of 5-fluorouracil, folinic acid and oxaliplatin. Treatment of metastatic colorectal carcinoma with FOLFOX is part of the therapeutic standard worldwide approved by relevant regulatory authorities and established in clinical practice. The additional use of 5-fluorouracil and folinic acid as part of the FOLFOX regimen improves the treatment results. In the proposed combination of the ultrasound-activated prodrug with other drugs according to the FOLFOX regimen, the possible side effects are limited by the selective accumulation in the tumor microenvironment and by the fact that the platinum(IV) complex is inactive in healthy tissue.

According to a further preferred embodiment of the invention, the molar ratio of components a) to b) is from 1 : 4 to 20 : 1, in particular from 1 : 2 to 10 : 1 and most preferably from 1 : 1 to 8 : 1.

Most preferably, component a) is present three times more than component b). As component a), the platinum complex is the therapeutically active agent, a largest possible share of component a) in the molecule is desirable. The order of components a) and b) within the polymeric prodrug nanoparticle is stochastically distributed regardless of the molar ratio.

A further preferred embodiment of the present invention is characterized in that the polymeric prodrug nanoparticle has a molecular weight of at least 5,000 g/mol, in particular at least 8,000 g/mol, preferably at least 10,000 g/mol.

This preferred molecular weight ensures that the polymeric prodrug nanoparticle is both stable and able to readily access the tumor tissue.

A preferred further embodiment of the invention is characterized by the fact that the prodrug can be activated to the active drug form by means of ultrasound. Particularly preferable the platinum(IV) of the prodrug is converted intratumorally into its therapeutically active form.

The targeted irradiation of the tumor region is an effective way of ensuring that the prodrug remains inactive in tumor-free tissue. In other words, the prodrug remains stable and therefore inactive in healthy tissue. Due to the use of ultrasound radiation, even deep-seated tumors can be reached. This ensures non-invasive treatment of tumors and metastases. Ultrasound irradiation rapidly reduces the prodrug in the tumor tissue by splitting off the two carbamate ligands into the therapeutically effective analog compound. This enables temporal and spatial control of the treatment. After reduction, the platinum compound is present in its therapeutically active form.

The object of the present invention is also achieved by the above-mentioned formulation of the polymeric prodrug nanoparticle described above in the form of a solution for injection or infusion.

The formulation as a solution for injection or infusion offers the advantage that different application methods can be used. The formulation is particularly preferably injected intravenously or intraperitoneally. While oxaliplatin cannot be injected intravenously, as it is locally toxic when injected locally, and therefore has to be slowly infused, the polymeric prodrug nanoparticle of the present invention can also be injected intravenously, which is another advantage of the present invention.

In the case of intravenous infusion or injection, the formulation is highly bioavailable. The formulation reliably reaches the desired target site via the blood vessels. In the case of intraperitoneal infusion or injection, the formulation acts locally with a high degree of effectiveness. This is particularly advantageous in the treatment intraperitoneal metastases.

The object of the present invention is also achieved by the aforementioned use of the polymeric prodrug nanoparticle described above or the formulation described above in cancer therapy, in particular for the treatment of tumor tissue and metastases. Tumor cells can spread into other tissues and organs and form metastases. For example, metastases can develop in the liver, the lungs, the non-regional lymph nodes or the peritoneum. Of these metastases, metastases in the lungs, liver and other regionally limited metastases, even if they are localized deeper in the body, can be treated particularly well with the polymeric prodrug nanoparticles according to the invention. It is particularly preferable to use the prodrug nanoparticle in conjunction with ultrasound irradiation of the tumor tissue to be treated. By irradiating, only the tumor tissue to be treated with ultrasound, the prodrug is only activated to the active substance there. In the non-irradiated areas, the prodrug remains in its inactive form, so that healthy tissue remains unaffected, and undesirable side effects are reduced. According to the present invention, treating tumors and its metastases, in particular in the liver, the lungs, the non-regional lymph nodes or the peritoneum, comprises a first step of administering to the patient the polymeric prodrug nanoparticle in a suitable form, in particular as an infusion. The tumor site is then selectively irradiated with ultrasound. The preferred frequency (or wavelength) of the ultrasound is from 0,1 MHz to 10 MHz, in particular from 1 MHz to 3 MHz.

The polymeric prodrug nanoparticles can be used for treating any kind of cancer. It is particularly suitable for treating carcinomas, sarcomas, lymphomas, melanomas and germ cell tumors, and their respective metastases. They are used for the non-invasive treatment of cancer and may be used for repeated treatment. The treatment may be curative or palliative. It can be used for treating the primary tumor as well as its metastases. It is typically used for a targeted local cancer treatment. The treatment using the polymeric prodrug nanoparticle according to the invention can very well be controlled timewise, as the reaction is initiated by radiation.

A particularly preferred application is the treatment of tumor tissue, in particular colorectal carcinoma tissue and the metastases of the carcinoma. Malignant tumor tissues e. g. colorectal carcinomas in particular form metastases in the liver, the lungs, the non-regional lymph nodes or the peritoneum. Of these metastases, metastases in the lungs, liver and other regionally limited metastases, even if they are localized deeper in the body, can be treated particularly well with the prodrug nanoparticles according to the invention.

The polymeric prodrug nanoparticle according to the present invention offers a reliable therapeutic approach in cancer therapy. When using the polymeric prodrug nanoparticle, already established therapeutic methods are applied, which lead to a considerably improved tolerability for the patient due to the activatable/reducible prodrug. This offers the advantage that, in contrast to conventional chemotherapies, its use has fewer effects on healthy tissue. The patient has significantly fewer side effects and therefore an improved quality of life during treatment.

The object of the present invention is also achieved by the aforementioned method for the preparation of polymeric prodrug nanoparticles, comprising the steps of
(i) reacting the at least one platinum(IV) complex and the at least one sonosensitizer with the linker for at least 1 hour, preferably 1 to 24 hours, particularly preferably 1 to 10 hours, and then
(ii) reacting the compound from step (i) with the compounds having a hydrophilic group, obtaining the polymeric prodrug nanoparticles.

This method of preparation is a simple way of ensuring that the components are stable and covalently bonded. The reaction is performed in an aqueous solution and at room temperature. As the compounds with hydrophilic groups are added at the final step of the reaction, they can only bind at the terminal ends and lead to the self-assembling nanoparticle structure at this point.

The subject matter and the embodiments of the present invention are further illustrated in the drawing which serve to visualize aspects of the examples following hereinafter and in which

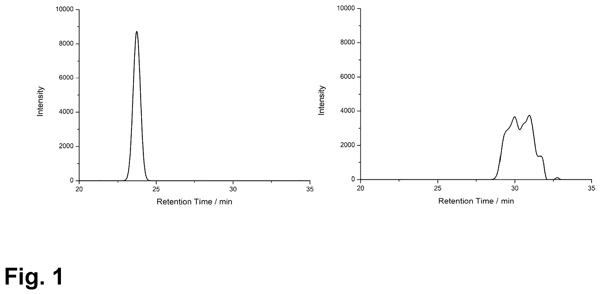

Figure 1 is a gel permeation chromatography chromatogram of the polymer upon incubation in dimethylformamide in the dark, i. e. without irradiation, for 24 h (left) or exposure to ultrasound radiation (right).

### Experimental

### Preparation of Hemin 6,7-bis-N-hydroxysuccinimide ester

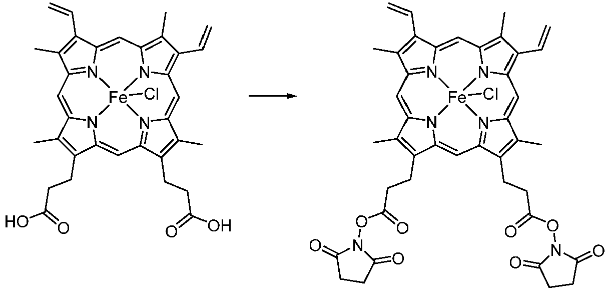

Hemin (0.39 mmol) was dissolved in dry dimethylformamide (5 mL) and cooled down to 0 °C. 1-Ethyl-3-(3-dimethylaminopropyl)carbodiimide (EDC-HCl) (0.9 mmol) was added to the reaction mixture and stirred for 1h at 0 °C. N-Hydroxysuccinimide (0.9 mmol) was added at the same temperature and stirred at room temperature overnight. After this time, the reaction mixture was filtered and extracted with acetonitrile (3 × 4 mL). The filtrate and the extract were combined and reduced to a volume of approximately 1 mL in vacuuo. The precipitated side product was removed by filtration and 50 mL of diethylether were added in small portions. The dark brown precipitated solid was filtered off and washed with diethylether (3 × 20 mL) and dried overnight in vacuum. Yield: 307 mg, 93%. Rf 0.79. ESI-MS (positive mode) for C42H38CIFeN6O8 m/z [M-Cl]+·O2 Calcd: 842.2, Found: 842.1.

### Preparation of Hemin 6,7-bis-N-(2-hydroxyethyl)amide

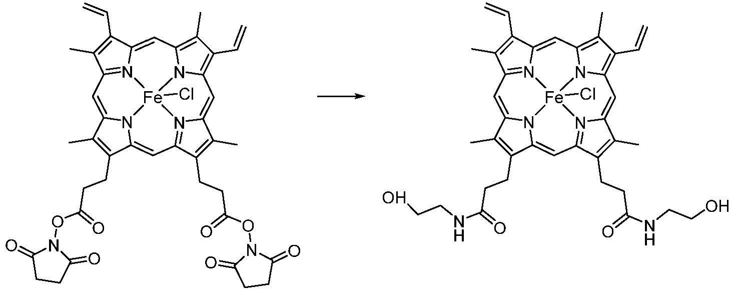

Hemin 6,7-bis-N-hydroxysuccinimide ester (0.05 mmol) and 2-aminoethan-1-ol (0.125 mmol) were dissolved in dimethylformamide (3 mL) and stirred at room temperature overnight. After this time, the solution was concentrated to approximately 1 mL and 5 mL of an aqueous solution of HCl (0.1 M) was added. Upon addition, the formed precipitate was isolated by filtration on filter paper and washed with water until neutral pH. Then, the filter paper with the residue was dried overnight in vacuum. The residue was redissolved in methanol and the volume was concentrated to approximately 1 mL. Then, 50 mL diethylether was added and the product re-precipitated as dark brown solid. The suspension was centrifuged for 5 minutes at 11000 rpm and washed with 30 mL diethylether. The solvent was decanted off and the collected product was dried overnight at room temperature. Yield: 27 mg, 73%. Rf 0.82. ESI-MS (positive mode) for C39H44ClFeN6O3 m/z [M-Cl]+ Calcd: 702.3, Found: 702.3.

### Oxaliplatin(IV) ([Pt(diaminocyclohexan)(oxalato)(OH)₂])

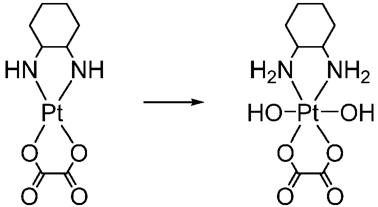

Oxaliplatin (0.39 mmol) was suspended in hydrogen peroxide (30% w/v, 2.0 mL). The mixture was stirred at 40 °C overnight until the solution was clear. After cooling down to room temperature, the product precipitated. The crude product was washed 3 × with acetone and dried under reduced pressure. Yield: 88 mg, 52%.¹H-NMR (400 MHz, (CD₃)₂SO): δ = 10.19 (s, 2H), 7.60 (s, 2H), 6.8 (s, 2H), 2.01-1.98 (d, 2H), 1.49-1.42 (m, 4H)1.24 (s, 2H), 1.11-1.08 (m, 2H) ppm.

### Preparation of Nanoparticles

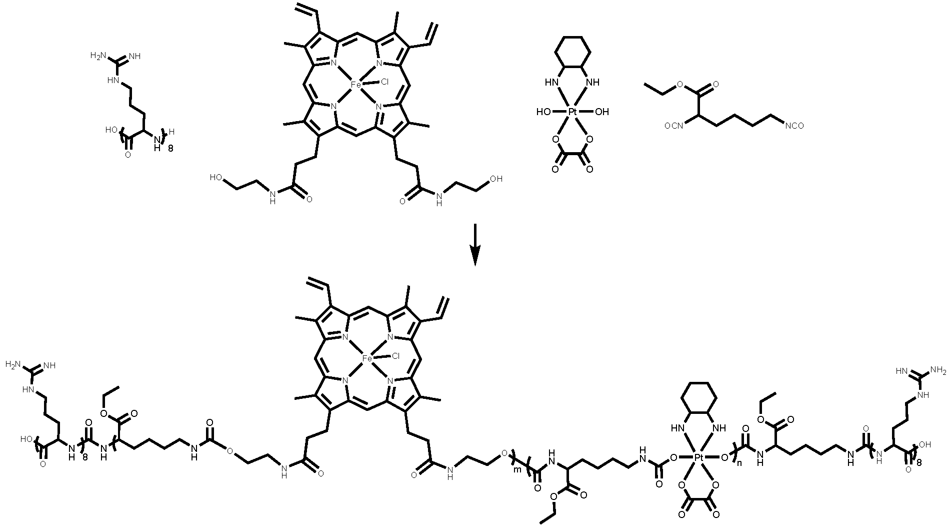

[Pt(diaminocyclohexan)(oxalato)(OH)₂] (0.06 mmol), Hemin 6,7-bis-N-(2-hydroxyethyl)amide (0.02 mmol), and ethyl 2,6-diisocyanatohexanoate (0.18 mmol) were suspended in dry dimethylformamide (10 mL) and the mixture stirred at room temperature for 1 h. After this time, (arginine)₈-NH₂ (0.02 mmol) was added to the reaction mixture and the mixture stirred at room temperature for 1 h. The reaction mixture was added into deionized water (25 mL). The solution was further purified by dialysis (MWCO: 3500 Da) for 72 h.

### 2. Photophysical Activation

The ability to reduce the platinum(IV) prodrug was assessed upon incubation of the platinum containing polymer in dimethylformamide and exposure to ultrasound radiation (1 MHz, 50% duty cycle, 1.5 W/cm², 30 min). The solution was afterwards analyzed by gel permeation chromatography. The results show that upon incubation of the polymer in water for up to 24 h, no decomposition was observed. In contrast, upon exposure to ultrasound radiation the decomposition of the polymer into small oligomeric and molecular compounds was observed (Figure 1).

For insight into the decomposition of the nanoparticles, the platinum containing nanoparticles were kept in the dark, i. e. without irradiation, or exposed to ultrasound radiation (1 MHz, 50% duty cycle, 1.5 W/cm², 30 min) in an aqueous solution. The intact structure of the nanoparticles was analyzed by dynamic light scattering. The results show that upon incubation of the polymer in water for up to 24 h, no decomposition was observed. In contrast, upon exposure to ultrasound radiation the decomposition of the nanoparticles due to the lack of any signal for nanostructured compounds was observed.

### 3. Biological Activity

6 × 10³ cells were seeded onto 96-well plates and allowed to adhere overnight. The cells were treated with increasing concentrations of the compound which was diluted in cell media to a total volume of 200 µL. The cells were incubated with the compound for 4 h, after this time washed, and the media refreshed. The cells for the dark group were grown for an additional 44 h at 37 °C. The cells for the ultrasound group were exposed to ultrasound radiation (1 MHz, 50% duty cycle, 1.5 W/cm², 5 min) with an Intelect Mobile 2 Ultrasound Emitter (DJO Global) and afterwards grown for an additional 44 h at 37 °C. After this time, the culture media was substituted with PBS buffer containing 3-(4,5-dimethylthiazol-2-yl)-2,5-diphenyltetrazolium bromide (MTT) with a final concentration of 12 µM. The cells were incubated for 2 h at 37 °C and 10 % CO₂ and the DPBS-MTT mixture was replaced by 200 µL DMSO. Using a 620 nm reference wavelength, the absorption of the formazan was measured at 550 nm with an Infinite M Nano plus Microplate reader (Tecan).

**Table 1. Cytotoxicity (in µM) of oxaliplatin and NP in the dark, i. e. without irradiation, or upon exposure to ultrasound radiation in mouse colon carcinoma (CT-26) and human breast cancer (MCF-7) cells. The mentioned concentrations correspond to the amount of the platinum complex.**

| | CT-26 | | MCF-7 | |
|---|---|---|---|---|
| | dark | ultrasound | dark | ultrasound |
| Nanoparticles | >100 | 8.5 ± 1.3 | >100 | 11.3 ± 1.2 |
| Oxaliplatin | 21.5 ± 3.6 | 19.7 ± 2.7 | 22.6 ± 2.7 | 21.8 ± 2.8 |

Unlike oxaliplatin, the prodrug nanoparticles of the present invention exhibit a very low toxicity in the dark, i. e. in the non-activated state, even for high concentration. As activation is only effected in the tumor, while the prodrug remains inactive in other, i. e. healthy, tissue, no toxic effect will be provoked in said healthy tissue.

## Claims

1. Polymeric prodrug nanoparticle for the targeted accumulation and activation in tumor tissue, consisting of the following monomers:
a) at least one platinum(IV) complex and
b) at least one sonosensitizer,
and
c) a compound having a hydrophilic group providing this hydrophilic group at each terminal end of the polymeric prodrug nanoparticle,
**characterized in that**
a), b) and c) are each covalently bonded to one another by means of a linker and a) and b) are arranged stochastically distributed within the polymeric prodrug nanoparticle.

2. Polymeric prodrug nanoparticle according to claim 1, **characterized in that** the general chemical structure of the polymeric product nanoparticle is a compound of the formula: 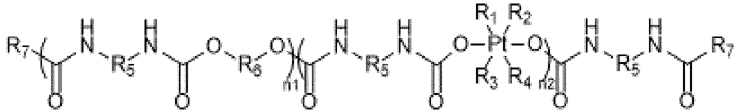 wherein:
R₁, R₂, R₃ and R₄ are independently selected from the group consisting of ammonia, hydroxide, halide, particularly chloride, oxalate, diamines, dicarboxylate and glycolate, optionally R₁ and R₂ are linked to form a first bidentate ligand, and/or R₃ and R₄ are linked to form a second bidentate ligand;
R₅ is the linker
R₆ is b),
R₇ is c) and
n₁, n₂ are 1 to 50, particularly 1 to 10, preferably 3 to 8.

3. Polymeric prodrug nanoparticle according to claim 1 or 2, **characterized in that** the at least one platinum(IV) complex is a platinum(IV) derivative of cisplatin, oxaliplatin, carboplatin, nedaplatin, lobaplatin or heptaplatin, wherein cisplatin, oxaliplatin and carboplatin are particularly preferred.

4. Polymeric prodrug nanoparticle according to one of claims 1 to 3, **characterized in that** the at least one sonosensitizer is a cyanine or a tetrapyrrole or a metal complex thereof, preferably a heme or a heme compound, in particular selected from the group consisting of hemoglobin, hemin b and hemin a, optionally containing one or particularly two spacer molecule substituents.

5. Polymeric prodrug nanoparticle according to one of claims 1 to 4, **characterized in that** the linker is a diisocyanate.

6. Polymeric prodrug nanoparticle according to one of claims 1 to 5, **characterized in that** the linker has a branched or unbranched alkyl radical on the functional group, the main chain of the alkyl radical having three to ten carbon atoms.

7. Polymeric prodrug nanoparticle according to one of claims 1 to 6, **characterized in that** the compounds having a hydrophilic group are PEG groups, peptides, proteins, antibodies, aptamers or small molecules.

8. Polymeric prodrug nanoparticle according to one of claims 1 to 7, **characterized in that** the compounds having a hydrophilic group are tumor antigen-binding or tumor-associated antigen-binding domains, in particular domains directed against VEGFR2.

9. Polymeric prodrug nanoparticle according to one of claims 1 to 8, **characterized in that** the ratio of components a) to b) is from 1 : 4 to 20 : 1, in particular from 1 : 2 to 10 : 1 and most preferably from 1 : 1 to 8 : 1.

10. Polymeric prodrug nanoparticle according to one of claims 1 to 9, **characterized in that** the polymeric prodrug nanoparticle is additionally loaded with at least one of at least one further antitumoral substance and at least one drug transporter inhibitor.

11. Polymeric prodrug nanoparticle according to one of claims 1 to 10, **characterized in that** the polymeric prodrug nanoparticle has a molecular weight of at least 5,000 g/mol, in particular at least 8,000 g/mol, preferably at least 10,000 g/mol.

12. Polymeric prodrug nanoparticle according to one of claims 1 to 11, **characterized in that** the prodrug can be activated to the active drug by means of ultrasound.

13. Polymeric prodrug nanoparticle according to one of claims 1 to 12, **characterized in that** the platinum(IV) of the prodrug is converted intratumorally into its therapeutically active form.

14. Formulation of the polymeric prodrug nanoparticle according to one of claims 1 to 13 in the form of an infusion or injection solution.

15. Use of the polymeric prodrug nanoparticle according to one of claims 1 to 14 or the formulation according to claim 12 in cancer therapy, in particular for the treatment of tumor tissue.

16. Use according to claim 15, **characterized in that** the use takes place in conjunction with ultrasound irradiation of the tumor tissue to be treated.

17. Method for the preparation of polymeric prodrug nanoparticles according to one of claims 1 to 13, comprising the steps of
(i) reacting the at least one platinum(IV) complex and the at least one sonosensitizer with the linker for at least 1 hour, preferably 1 to 24 hours, particularly preferably 1 to 10 hours and then
(ii) reacting the compound from step (i) with the compounds having a hydrophilic group, obtaining the polymeric prodrug nanoparticles.
